(19) Europäisches Patentamt
European Patent Office
Office européen des brevets

(11) Publication number: **0 401 903 A2**

(12) # EUROPEAN PATENT APPLICATION

(21) Application number: **90201380.4**

(22) Date of filing: **30.05.90**

(51) Int. Cl.5: **A61K 31/165, A61K 31/17, A61K 31/18**

(30) Priority: **08.06.89 US 363239**
**08.09.89 US 404711**

(43) Date of publication of application:
**12.12.90 Bulletin 90/50**

(84) Designated Contracting States:
**AT BE CH DE DK ES FR GB GR IT LI LU NL SE**

(71) Applicant: **THE PROCTER & GAMBLE COMPANY**
**One Procter & Gamble Plaza**
**Cincinnati Ohio 45202(US)**

(72) Inventor: **Martodam, Raymond Richard**
**2031 Parkhurst Court**
**Cincinnati, OH 45224(US)**
Inventor: **Mizoguchi, Haruko**
**5740 Auberger Drive**
**Fairfield, OH 45014(US)**

(74) Representative: **Suslic, Lydia et al**
**Procter & Gamble European Technical Center N.V. Temselaan 100**
**B-1853 Strombeek-Bever(BE)**

(54) **The use of vanilloids for the treatment of respiratory diseases or disorders.**

(57) The present invention provides methods of treating respiratory diseases or disorders in humans and lower animals by treatment with natural and synthetic vanilloid compounds, and the pharmaceutically-acceptable salts thereof.

This invention further relates to pharmaceutical compositions containing said natural and synthetic vanilloid compounds useful to treat the respiratory symptoms associated with respiratory diseases or disorders.

EP 0 401 903 A2

# THE USE OF VANILLOIDS FOR THE TREATMENT OF RESPIRATORY DISEASES OR DISORDERS

## TECHNICAL FIELD

This application relates to the use of vanilloid compounds for the treatment of respiratory tract diseases or disorders.

## BACKGROUND OF THE INVENTION

The present invention involves a novel use for natural and synthetic vanilloid compounds. The following are non-limiting examples of such vanilloid compounds, and references in which they are disclosed; all of the following references are hereby incorporated herein in their entirety by reference: capsaicin (trans-8-methyl-N-vanillyl-6-nonenamide) and "synthetic" capsaicin (N-vanillylnonanamide) in U.S. Patent 4,313,958, LaHann, issued February 2, 1982; capsaicin in Yaksh, et al, Science, 206, pp 481-483 (1979); capsaicin in Jancso, et al, Naunyn-Schmiedeberg's Arch. Pharmacol., Vol. 311, pp 285-288 (1980); capsaicin in Holzer et al, Eur. J. Pharm. Vol. 58, pp. 511-514 (1979); 3-hydroxyacetanilide in U.S. Patent 4,238,508, Nelson, issued December 9, 1980; hydroxyphenylacetamides in European Patent Application 0089710, LaHann, et al, published September 28, 1983; N-vanillyl sulfonamides in U.S. Patent 4,401,663, Buckwalter, et al, issued August 30, 1983, hydroxyphenyl-acetamides in U.S. Patent 4,424,205, LaHann, et al, issued January 31, 1984; N-(3- or 4- hydroxy or 3,4-dihydroxybenzyl) carbamates in U.S. Patent 4,443,473, Buckwalter, et al, issued April 17, 1984; N-[(substituted phenyl) methyl]-cis-monounsaturated alkenamides in U.S. Patent 4,493,848, LaHann, et al, issued January 15, 1985; N-(3-methoxy-4-hydroxybenzyl and phenyl) ureas and thioureas in U.S. Patent 4,460,602, Buckwalter, et al, issued July 17, 1984; N-vanillylureas in European Patent Application 0068590, Buckwalter, et al, published January 5, 1983; N-[(substituted phenyl)methyl] alkynamides in U.S. Patent 4,532,139, Janusz, et al, issued July 30, 1985; methylene substituted N-[-(substituted phenyl)methyl] alkanamides in U.S. Patent 4,544,668, Janusz, et al, issued October 1, 1985; N-[(substituted phenyl) methyl]-diunsaturated amides in U.S. Patent 4,544,669, LaHann, et al, issued October 1, 1985; monoalkenamides in U.S. Patent 4,564,633, LaHann, et al, issued January 14, 1986; substituted phenylacetic acid esters in British Patent Specification 2,168,974, Loomans, et al, published July 2, 1986; N-(substituted alkyl)alkanamides and thioamides in British Patent Specification 2,168,976, Loomans, et al, published July 2, 1986; substituted aromatic-araalkanamides in British Patent Specification 2,168,975, Janusz et al, published July 2, 1986; and amino-ethyl compounds in European Patent Application 0282127, Gardner et al, published September 14, 1988.

Vanilloid compounds have been generally disclosed in the above references to have analgesic, anti-irritant and anti-inflammatory activity.

The effects of capsaicin on the respiratory tract of humans and other animals has been disclosed in a number of references. The following references disclose application of capsaicin to the nasal mucosa. Geppetti, P., B. M. Fusco, S. Marabini, C. A. Maggi, M. Fanciullacci, and F. Ficuteri, "Secretion, Pain and Sneezing Induced by the Application of Capsaicin to the Nasal Mucosa in Man", Br. J. Pharmacol. Vol. 93, (1988), pp. 509-514, discloses a burning, pungent, pain sensation and copious flow of nasal secretion upon topical application of capsaicin. Desensitization and time-dependent recovery, as well as induced sneezing, is also disclosed. Marabini, S., G. Ciabatti, G. Polli, B. M. Fusco, P. Geppeti, C. A. Maggi, M. Fanciullacci, F. Sicuteri, "Effect of Topical Nasal Treatment with Capsaicin in Vasomotor Rhinitis" Regulatory Peptides, Vol. 22, No. 1/2, (July 1988), p. 121, discloses that topical nasal treatment with capsaicin effects vasomotor rhinitis by desensitizing patients affected by chronic vasomotor rhinitis after 4-5 days of treatment. Saria, A., and G. Wolf, "Beneficial Effects of Topically Applied Capsaicin in the Treatment of Hyperreactive Rhinopathy", Regulatory Peptides, Vol. 22, No. 1/2, (July 1988), p.167, discloses that capsaicin causes selective blockage of primary afferent nerves containing tachykinin (TK) and other peptides such as calcitonin gene related peptide after repeated topical or systemic application. Hypersecretion, decreased nasal patency and sneezing attacks of severe intensity are disclosed as completely disappearing in eight of nine patients after the fifth treatment. Capsaicin-sensitive neurons are disclosed as being involved in the pathogenesis of hyperreactive (hyperreflectoric) rhinopathy or vasomotor rhinitis. Wolf, G., D. Loidolt, A. Saria, R. Gamse, "Anderungen des nasalen Volumsstromes nach lokaler Applikation des Neuropeptides

Substanz-P und von Capsaicin", Laryng. Rhinol. Atol., Vol. 66, (1987) pp. 412-415, discloses that the local or systemic application of capsaicin causes the release of substance P. Further application causes desensitization of the polynodal nociceptors and selective degeneration of afferent C-fibers. A decrease in air flow volume and a hypersecretion of the nasal mucosa with increased tear flow is disclosed. Lundblad, L., and J. M. Lundberg, "Capsaicin Sensitive Sensory Neurons Mediate the Response to Nasal Irritation Induced by the Vapour Phase of Cigarette Smoke", Toxicology, Vol. 33, (1984) pp. 1-7, discloses that systemic capsaicin pretreatment abolished nose-wiping behavior on smoke exposure in awake guinea pigs. Local pretreatment of the nasal mucosa with capsaicin also is disclosed as significantly decreasing the number of smoke induced nose-wipings. It is postulated that smoke-induced irritation as indicated by nose-wiping, is primarily due to activation of capsaicin-sensitive sensory nerves in the nasal mucosa by vapor phase components.

Lundblad, L., J. M. Lundberg, E. Brodin and A. Anggard, "Origin and Distribution of Capsaicin-Sensitive Substance P-Immunoreactive Nerves in the Nasal Mucosa", Acta Otolaryngol, Vol. 96, (1983), pp. 485-493, discloses that capsaicin pretreatment of guinea pigs and rats resulted in a selective loss of SP-IR (substance P-immunoreactivity) nerves in the nasal mucosa and sphenopalatine ganglion, while the parasympathetic nerves were still present.

Relationships between capsaicin, mucus secretion and Substance P have been disclosed in other references. Lundblad, L., A. Saria, J. M. Lundberg and A. Anggard, "Increased Vascular Permeability in Rat Nasal Mucosa Induced by Substance P and Stimulation of Capsaicin-sensitive Trigeminal Neurons, Acta Otolaryngol, Vol. 96, (1983) pp. 479-484, discloses that hemogenic irritation of the nasal mucosa by capsaicin induces edema, probably via a local axon reflex inducing release of Substance P. Capsaicin-sensitive Substance P containing afferents in the nasal mucosa were also postulated to be involved in the pathogenesis of nasal congestion seen in various types of rhinitis. Mizoguchi, H. and R. Hicks, "Effects of Neurokinins on Vascular Permeability in Guinea Pigs", April 1987 FASEB Abstract Form, The Procter & Gamble Company, Cincinnati, Ohio 45247, (1986), discloses increased vascular permeability upon intravenous administration of Substance P. A synthetic analogue of capsaicin, administered intratracheally, is also disclosed to produce a dose-related increase in vascular permeability. Shimura, S., T. Sasaki, H. Okayama, H. Sasaki and T. Takishima, "Effect of Substance P on Mucus Secretion of Isolated Submucosal Gland from Feline Trachea", J. Appl. Physiol., Vol. 63, No. 2, (1987), pp. 646-653, discloses dose-dependent increases in the contractile response of the feline trachea due to Substance P. Capsaicin is disclosed to have induced a tension of a magnitude similar to that of Substance P. Capsaicin is also disclosed as inducing an acute release of Substance P immunoreactivity from peripheral sensory nerve endings. Coles, S., K. Neill and L. Reid, "Potent Stimulation of Glycoprotein Secretion in Canine Trachea by Substance P", J. Appl. Physiol.:Respirat. Enbrion. Exercise Physiol., Vol. 57, No. 5, (1984), pp. 1323-1327, discloses that Substance P may be important in mediating irritant-induced mucus hypersecretion. Lundblad, L., A. Anggard and J. M. Lundberg, "Effects of Antidromic Trigeminal Nerve Stimulation in Relation to Parasympathetic Vasodilation in Cat Nasal Mucosa", Acta Physiol. Scand., Vol. 119, (1983), pp. 7-13, discloses that local intraarterial infusions of Substance P, vasoactive intestinal polypeptide (VIP) and acetylcholine cause a dose-dependent vasodilatation of the nasal mucosa. Local infusions of capsaicin are disclosed as causing a marked long-lasting biphasic vasodilation which is atropine and hexamethonium resistant. The initial vasodilation caused by capsaicin and the prolonged vasodilatory response to capsaicin are disclosed as likely being caused by different mechanisms. No clear cut inhibition of the trigeminal response was seen using the short time infusions in this study (page 11).

Capsaicin and the sneeze reflex have been disclosed in a number of references. Lundblad, L., E. Brodin, J. M. Lundberg and A. Anggard, "Effects of Nasal Capsaicin Pretreatment on Sneezing Reflexes, Neurogenic Plasma Extravasation, Sensory and Sympathetic Neurons", Acta Otolaryngol (Stockh), Vol. 100, (1985), pp. 117-127, discloses that the sneezing response to local application of capsaicin, but not that to nicotine, was reduced or abolished by capsaicin pretreatment and cryosurgery, while the response to tactile stimulation was unaffected. Lundblad, L., J. M. Lundberg and A. Anggard, "Local and systemic capsaicin pretreatment inhibits sneezing induced by certain irritants", Naunyn-Schmiedeberg's Arch. Pharmacol., Volume 326, (1984), pp. 254-261, discloses that local application of capsaicin reduced or abolished sneezing responses to capsaicin and formalin, The sneezing response to mechanical stimuli after local application of capsaicin was not affected. The response to nicotine was also reported reduced following local pretreatment with capsaicin. Lundblad, L., "Protective reflexes and vascular effects in the nasal mucosa elicited by activation of capsaicin-sensitive substance P-immunoreactive trigeminal neurons", Acta Physiologica Scandinavica, Supplementum 529, (1984), pp. 1-42, discloses capsaicin pretreatment of the nasal mucosa causes desensitization against chemical irritation caused by capsaicin, formalin, ether, nicotine or cigarette smoke. The nicotine induced sneezing was mainly resistant to capsaicin pretreatment,

while formalin or capsaicin induced sneezing was decreased by capsaicin pretreatment. Irritation induced by tactile stimulation of the nasal mucosa was disclosed to be unchanged by capsaicin pretreatment.

Behavioral, cutaneous itching and allergy reactions to capsaicin are disclosed in the following references. Gamse, R., A. Saria, J. M. Lundberg and E. Theodorsson-Norheim, "Behavioral and Chemical Changes after Intracisternal Capsaicin Treatment of the Guinea Pig", Neuroscience Letters, Vol. 64, (1986), pp. 287-292, discloses that capsaicin pretreatment intracisternally or intraperitoneally in the guinea pig resulted in behavioral responses to the irritation effects of capsaicin applied to the eye or nose; to ether vapor; and to hot water applied to the forepaw or ear. Pretreatment with capsaicin also abolished the increased vascular permeability in the nasal mucosa induced by irritants, and it depleted the substance P-immuno reactivity, i.e., the assumed mediator of vascular permeability. Toth-Kasa, I., G. Jancso, A. Bognar, S. Husz, F. Obal Jr., "Capsaicin Prevents Histamine-induced Itching, Int. J. Clin. Pharm. Res., Vol. 6, No. 2, (1986), pp. 163-169, discloses that topical pretreatment of the human skin with capsaicin resulted in a reversible marked reduction or abolition of the axon reflex flare, but did not influence whealing on histamine-induced pruritus. Itching was also strongly diminished or even abolished, provided that the flare response was completely blocked. Lundblad, L., J. M. Lundberg, A. Anggard and O. Zetterstrom, "Capsaicin-sensitive Nerves and the Cutaneous Allergy Reaction in Man", Allergy, Vol. 42, (1987), pp. 20-25, discloses that local cutaneous capsaicin pretreatment affects the cutaneous triple response reaction (itching, flare or vasodilation and wheal or protein extravasation) in man. Acute exposure of the human skin to capsaicin caused a burning sensation and a clear-cut flare reaction, but no wheal response. Upon repeated administration these local reactions to capsaicin disappeared. Jancso, G., E. Kiraly and A. Jancso-Gabor, "Pharmacologically Induced Selected Degeneration of Chemosensitive Primary Sensory Neurones", Nature, Vol. 270, (December 1977), pp. 741-743, discloses that capsaicin, after an initial violent stimulation, renders sensory nerve endings in the skin and mucous membranes of different species insensitive to chemical pain stimuli for a long time. In the rat, marked systemic desensitization greatly inhibits the neurogenic inflammation induced either by pain-producing chemical irritants or by antidromic electrical stimulation of sensory nerves.

## Respiratory Tract Diseases and Disorders

The respiratory system in man reacts to foreign substances through a complex mechanism involving cough, sneeze, increased nasal secretion or rhinorrhea and congestion. Cough is a protective reflex that can expel secretions, exudates, transudates or extraneous materials from the respiratory tract. Antitussive medications inhibit or supress cough by acting on either the central or peripheral components of the cough reflex. Many agents, such as codeine and dextromethorphan, suppress the cough reflex by depressing the medullary cough center or associated higher centers. Other antitussives act as mild analgesic or anesthetics on the repiratory mucosa. Demulcents are useful against cough arising above the larynx, acting by forming a protective coating over the irritated pharyngeal mucosa. Local anesthetics such as benzocaine, cyclaine and tetracaine are used to inhibit the cough reflex under special circumstances; e. g., before bronchoscopy or bronchography. Other antitussives such as humidifying aerosols and steam inhalation exert their effects by demulcent action and by decreasing the viscosity of bronchial secretions. Expectorants also produce their antitussive effect by decreasing the viscosity of broncial secretions, often producing increased bronchial secretions through reflex irritation of the bronchial mucosa.

Rhinorrhea and congestion are also present in a number of respiratory diseases or disorders. Bronchial asthma can occur secondarily to a variety of stimuli. Persons manifestings this disorder often have hyperreactive bronchi, sometimes with associated bronchoconstriction. Asthmatic attacks are characterized by narrowing of large and small airways due to spasm of bronchial smooth muscle, edema and inflammation of the bronchial mucosa and production of tenacious mucus. Asthma precipitated by allergens is often termed "extrinsic asthma" and accounts for about 10 to 20% of adult asthmatics. 30 to 50% of asthmatic episodes appear to be triggered by non-allergenic factors (e.g., infection, irritants). These asthmatics are said to have nonallergic or "intrinsic asthma". In many persons, both allergenic and non-allergenic factors are significant. Persistent asthma and chronic asthmatic bronchitis sufferers also display chronic, productive cough.

Acute bronchitis may develop after nasopharyngeal, throat or tracheobronchial tree infections, with exposure to external elements and other agents as contributory factors. Sore throat followed by onset of cough usually signals onset of bronchitis, with initially dry nonproductive cough becoming mucoid or mucopurulent (productive) with the passage of a few hours or days. Chronic bronchitis is a condition associated with prolonged exposure to nonspecific bronchial irritants and accompanied by mucus hypersecretion and certain structural changes in the bronchi. Chronic bronchitis is also characterized clinically by

chronic productive cough.

Pain or itch is often associated with irritation of respiratory mucosa. Anesthetics may reduce such pain, but often results in loss of mechanical sensation (numbing) of the nasal and upper respiratory tract mucosa.

## Objects of the Present Invention

It is an object of the present invention to provide methods for treating respiratory diseases or disorders and the attendant discomfort often associated with respiratory diseases or disorders.

It is a further object of the present invention to provide methods for topically treating such respiratory diseases or disorders.

It is a still further object of the present invention to provide methods for treating intrinsic or extrinsic diseases or disorders such as intrinsic or extrinsic asthma.

It is a still further object of the present invention to provide methods of treatment which resolve clinical symptoms of respiratory diseases or disorders without causing loss of mechanical sensation (i.e., "numbing") in the respiratory tract or loss of olfactory competence.

It is a still further object of the present invention to provide methods of treatment which resolve clinical symptoms of respiratory diseases or disorders while also decreasing the pain or itch associated with irritation of respiratory mucosa.

## SUMMARY OF THE INVENTION

The present invention provides methods of treating respiratory diseases or disorders in humans and lower animals by treatment with natural and synthetic vanilloid compounds, and the pharmaceutically-acceptable salts thereof.

This invention particularly relates to methods of treatment whereby pharmaceutical compositions containing said natural and synthetic vanilloid compounds are locally or regionally applied to the respiratory tract to treat the respiratory symptoms associated with respiratory diseases or disorders.

## DETAILED DESCRIPTION OF THE INVENTION

The present invention relates to the use of capsaicin and the vanilloids to provide long-lasting relief from the symptoms associated with respiratory diseases or disorders. By applying capsaicin or the vanilloids to the nasal or respiratory mucosa of animals or man suffering from respiratory symptoms associated with respiratory diseases or disorders, such symptoms as cough, sneeze, throat pain and itch, rhinorrhea (mucous hypersecretion) and congestion are eliminated. The present invention further relates to pharmaceutical compositions containing natural or synthetic vanilloid compounds, useful to treat the respiratory symptoms associated with respiratory diseases or disorders.

Respiratory diseases or disorders that can be treated by the methods of the present invention include such diseases or disorders as the common cold; extrinsic or intrinsic asthma; chronic obstructive pulmonary disease including chronic bronchitis and bronchiolitis; diseases of the extrathoracic (upper) airways caused by viral infection; allergic rhinitis; vasomotor rhinitis; disorders associated with exogenous irritants such as tobacco smoke, smog, high levels of atmospheric $SO_2$ and noxious gases in the workplace; airways hyperreactivity; milk product intolerance; Loffler's pneumonia; emphysema; cystic fibrosis; bronchiectasis; pulmonary fibrosis; pneumoconiosis; collagen vascular disease; granulomatous disease; laryngitis; acute bronchitis; pharyngitis; pneumonia; pleuritis; persistent asthma and chronic asthmatic bronchitis.

More particularly, respiratory diseases or disorders that can be treated by the methods of the present invention include the common cold, extrinsic or intrinsic asthma; chronic bronchitis; diseases of the extrathoracic (upper) airways caused by viral infection; allergic rhinitis and vasomotor rhinitis; airways hyperreactivity; milk product intolerance; Loffler's pneumonia; emphysema; cystic fibrosis; bronchiectasis; pulmonary fibrosis; pneumoconiosis; collagen vascular disease; granulomatous disease; laryngitis and pharyngitis.

Even more particularly, respiratory diseases or disorders that can be treated by the methods of the present invention include the common cold, intrinsic asthma, chronic bronchitis, diseases of the extrathoracic (upper) airways caused by viral infection, allergic rhinitis, vasomotor rhinitis, laryngitis and pharyngitis.

Most particularly, respiratory diseases or disorders that can be treated by the methods of the present invention include intrinsic asthma; allergic or rhinitis, vasomotor rhinitis; or laryngitis and pharyngitis.

The term "substituted", as used herein for alkyl and aryl groups, means alkyl or aryl groups that can be mono- or polysubstituted, especially mono-, di-, or trisubstituted. Preferred substitutents are selected from the group consisting of halogen, (especially fluorine, chlorine or bromine) hydroxy, amino, cyano, thiol, aryl (especially phenyl or naphthyl), alkyl (preferably $C_1$-$C_6$ alkyl, more preferably methyl or ethyl), carboxylate, nitro, $-CF_3$ and $-OR^5$ wherein $R^5$ is an unsubstituted alkyl group having from about 1 to about 3 carbon atoms (especially methoxy and ethoxy).

The term "alkyl", as used herein, means carbon-containing chains which may be straight, branched, or cyclic; substituted or unsubstituted; and which may be saturated, monounsaturated (i.e., one double or triple bond in the chain), or polyunsaturated (e.g., two or more double bonds in the chain; two or more triple bonds in the chain; one or more double and one or more triple bonds in the chain). As used herein, saturated alkyl groups are referred to as "alkanyl"; unsaturated alkyl groups comprising double bonds in the chain are referred to as "alkenyl" (preferred are chains having the double bonds in the "Z" or "cis" geometric configuration); and unsaturated alkyl groups comprising triple bonds in the chain are referred to as "alkynyl". The designation of geometric configurations for any double bonds present in compounds of the present invention utilizes the art-known nomenclature "Z" and "E", and is fully described in Morrison and Boyd, Organic Chemistry, Third Edition (Allyn and Bacon, Inc., Boston; 1973), pp. 131-133 and 148-151; and March, Advanced Organic Chemistry, Second Edition (McGraw-Hill Book Company, New York; 1977), pp. 86-124; the disclosures of both these references being incorporated herein by reference in their entirety.

The term "short chain alkyl", as used herein, means $C_1$-$C_6$ alkyl chains which may be straight, branched or cyclic (preferably straight), saturated, monounsaturated or polyunsaturated (preferably saturated) and substituted or unsubstituted (preferably unsubstituted).

The terms "aryl" and "heteroaryl", as used herein, mean aryl or heteroaryl rings which may be mono-, di-, or tri-substituted or unsubstituted, preferably monosubstituted or unsubstituted. Additionally, heteroaryl rings comprise at least one oxygen, sulfur or nitrogen atom in the ring structure. Preferred aryls and heteroaryls include substituted or unsubstituted phenyl, naphthyl, pyridyl, pyrimidyl, imidazolyl, furanyl, thiophenyl, pyrazolyl, triazolyl, tetrazolyl, thiazolyl, triazinyl, pyrrolyl, indolyl and purinyl. More preferred aryls and heteroaryls include unsubstituted and substituted phenyl, pyridyl, imidazolyl, furanyl and thiophenyl. Most preferred aryl is unsubstituted or substituted phenyl. Preferred substituents include halogen,

The term "carboxylate", as used herein, means an organic carboxylic acid moiety (i.e., $-CO_2H$), and the salts (e.g., sodium, potassium, calcium, tetraethylammonium) and esters (e.g., methyl ester, ethyl ester) and amides (e.g., unsubstituted amide, N-methylamide, N,N-dimethylamide) thereof which are acceptable from a toxicity viewpoint for administration to humans or lower animals.

The term "pharmaceutically-acceptable salts and amides", as used herein, means the compounds in their salt or amide form which have the same general pharmacological properties as the basic amino form from which they are derived, and which are acceptable from a toxicity viewpoint. Pharmaceutically-acceptable salts include ammonium salts derived from inorganic acids (e.g., HCl, HBr, $NaHSO_4$, $H_2CO_3$), and ammonium carboxylic acid salts derived from organic carboxylic acids (e.g., acetic acid; lactic acid, gluconic acid; citric acid; glucouronic acid; galactouronic acid; fumaric acid; gentisic acid; lactobionic acid; benzoic acid). Pharmaceutically-acceptable amides include those derived from organic carboxylic acids (e.g., acetic acid amides) including amino acids (e.g., glycine amides). Preferred are the ammonium carboxylic acid salts derived from organic carboxylic acids, especially the acetate and lactate salts.

The compounds useful in the present invention are natural and synthetic vanilloid compounds, and the pharmaceutically-acceptable salts thereof, having the general structure:

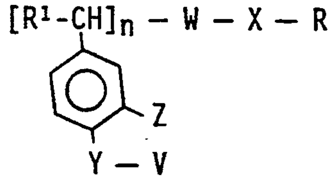

(1)

In structure (1) n = 0 or 1.

In structure (1), the -W-X- moiety is selected from -C(O)NH-, -C(S)NH-, -$S(O)_2$NH-, -NHC(O)O-, -NHC(S)O-, -NHC(O)NH-, and -NHC(S)NH-. Preferred -W-X- is selected from -C(O)NH-, -C(S)NH-, -NHC(O)NH-, -NHC(S)NH- and -$S(O)_2$NH-. More preferred -W-X- is selected from -C(O)NH-, -C(S)NH-, and -NHC(O)NH-. Most preferred -W-X- is -C(O)NH-. Either available bond of the -W-X- moiety may be bonded to the -R moiety, with the other bond being attached to the benzyl carbon atom, or directly attached to the benzene ring.

In structure (1), the -$R^1$ moiety is selected from hydrogen, hydroxy, alkyl esters of hydroxy having from about 1 to about 5 carbon atoms, alkyl having from about 1 to about 5 carbon atoms, and alkoxy having from about 1 to about 5 carbon atoms. Preferred -$R^1$ is selected from hydrogen, hydroxy, and methyl; most preferred -$R^1$ is hydrogen.

In structure (1), the -Z moiety is selected from hydrogen, hydroxy and methoxy; preferred -Z is selected from hydroxy and methoxy. Most preferred -Z is methoxy.

In structure (1), the -Y- moiety is selected from -O-, -S-, -$NR^4$-, -OC(O)-, -$OSO_3^-$- and -$OPO_3^=$- where -$R^4$ is selected from hydrogen and $C_1$-$C_4$ alkanyl. Preferred -Y- is selected from -O- , -S- and -NH-. More preferred -Y- is selected from -O- and -S-; most preferred -Y- is -O-.

In structure (1) the -V moiety is selected from hydrogen, short chain alkyl, and -$CR^2_2$-$CR^2_2$-$NH_2$. Preferred -V is selected from short chain alkyl, hydrogen and -$CR^2_2$-$CR^2_2$-$NH_2$. Even more preferred -V is selected from hydrogen and methyl, especially hydrogen. Also more preferred is -$CR^2_2$-$CR^2_2$-$NH_2$.

The -$R^2$ moieties are each independently selected from hydrogen; halogen; unsubstituted or substituted alkyl, the alkyl portion having from about 1 to about 6 carbon atoms; substituted or unsubstituted aryl or heteroaryl; and carboxylate; or two -$R^2$ moieties are covalently bonded to form a substituted or unsubstituted alkyl, heteroalkyl, aryl or heteroaryl ring having from about 3 to about 8 atoms, preferably 3-6, in the ring, including from 0 to about 3 heteroatoms. It is preferred that no more than two -$R^2$ are other than hydrogen. Preferred -$R^2$ substituents other than hydrogen include unsubstituted and substituted $C_1$-$C_6$ alkyl and unsubstituted and substituted phenyl.

It is preferred that at least one -$R^2$ on the alpha carbon atom (i.e., the carbon atom bonded directly to the Y moiety) be a hydrogen. Preferred also is all -$R^2$ being selected from hydrogen and hydroxyalkyl having from about 1 to about 5 carbon atoms, more preferably 5-hydroxypentyl, 2-hydroxybutyl or hydroxymethyl, especially hydroxymethyl. Preferred also is all -$R^2$ being selected from hydrogen and aminoalkyl having from about 1 to about 5 carbon atoms, more preferably 2-aminopentyl, 2-aminobutyl, aminomethyl or aminoethyl, especially aminomethyl or aminoethyl. Preferred also is all -$R^2$ being selected from hydrogen and substituted or unsubstituted aryl, especially phenyl or methylphenyl. Preferred -$R^2$ moieties which are aryls include phenyl, naphthyl, and substituted phenyl or naphthyl; most preferred being substituted or unsubstituted phenyl. Preferred -$R^2$ moieties which are arylalkyls are substituted, or preferably, unsubstituted. Preferred -$R^2$ moieties which are substituted arylalkyls are those where the substituent groups are independently selected from $C_1$-$C_4$ alkyl, $C_1$-$C_4$ alkoxy, halogen, hydroxy, amino, hydrogen and carboxy groups. Preferred also is all -$R^2$ being selected from hydrogen and alkyl having from about 1 to about 5 carbon atoms (especially methyl). Also preferred is at most only one -$R^2$ being other than hydrogen. Also preferred is all -$R^2$ being hydrogen.

Particularly preferred is where both -$R^2$ on the alpha carbon atom are hydrogen and both -$R^2$ on the beta carbon atom (the carbon atom bonded directly to the alpha carbon atom) are unsubstituted or substituted alkyl or are covalently bonded to form a substituted or unsubstituted alkyl or heteroalkyl ring having from about 3 to about 8 atoms, including from 0 to about 3 heteroatoms, in the ring. As used herein, "heteroatoms" means atoms other than carbon that can covalently bond to at least two other atoms and become part of a stable ring structure. Preferred heteroatoms are N, O and S. More preferred -$R^2$ on the beta carbon atom are unsubstituted or substituted $C_1$-$C_6$ alkyl, more preferably $C_1$-$C_4$ alkyl, more preferably still $C_1$-$C_2$ alkyl. Also preferred are the two -$R^2$ moieties on the beta carbon atom being covalently bonded to form a substituted or unsubstituted alkyl ring having from about 3 to about 6 carbon atoms, more preferably 3 or 4 or 5 carbon atoms in the ring. Preferred -$R^2$ alkyl moieties on the beta carbon atom are saturated or unsaturated having a single double or triple bond, more preferred is that both -$R^2$ on the beta carbon be unsubstituted or substituted alkanyl or covalently bonded to form an unsubstituted or substituted alkanyl ring. Preferred substituents of the -$R^2$ alkyl moieties on the beta carbon are hydroxy, amino, thiol and carboxylate, especially hydroxy and amino. More preferred is that all -$R^2$ alkyl moieties on the beta carbon being unsubstituted. More preferred still is that both -$R^2$ on the beta carbon atom are methyl or ethyl, especially methyl.

In structure (1) the -R moiety is a $C_1$-$C_{24}$ alkyl moiety which may be straight, branched or cyclic chain and may be saturated, monounsaturated, or polyunsaturated, substituted or unsubstituted.

Preferred -R moieties are straight and branched chain alkanyl, straight and branched chain monounsaturated alkyl, straight and branched chain diunsaturated alkyl, and straight and branched chain triunsaturated alkyl. More preferred -R moieties are mono- or diunsaturated or saturated, $C_6$-$C_{24}$ straight or branched chain alkyls. Also more preferred are $C_5$-$C_{11}$ straight chain alkyls. Even more preferred are mono- or diunsaturated alkenyls, or $C_6$-$C_{24}$ straight chain alkyls. Further preferred are monounsaturated cis-double bond $C_{11}$-$C_{24}$ straight chain alkenyls. Also further preferred are mono-unsaturated or saturated, $C_6$-$C_{10}$ straight chain alkyls. Even further preferred is mono-unsaturated cis-double bond $C_{14}$-$C_{23}$ straight chain alkenyls. Most preferred -R is 9-Z-octadecenyl. Such preferred -R moieties are preferably unsubstituted.

Other preferred -R moieties are arylalkyls having a $C_1$-$C_{12}$, more preferably $C_1$-$C_6$, most preferably $C_1$-$C_2$, alkyl portion which is preferably straight chain and also preferably alkanyl. The aryl portion is preferably unsubstituted or substituted phenyl. Preferred substituents include halogen, nitro, cyano, phenyl, benzyl, benzyloxy, trifluoromethyl, pormylamino, $C_1$-$C_{16}$ alkoxy and $C_1$-$C_4$ alkyl.

Preferred -R groups are as follows. For the methods of the present invention which use phenylacetic acid amide or thioamide derivatives, particularly the beta-aminoethoxy-substituted compounds having the general structure:

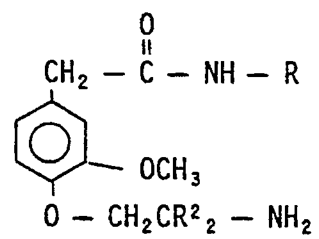,

the preferred -R groups are selected from n-hexanyl, n-heptanyl, n-octanyl, n-nonanyl, n-decanyl, n-undecanyl, n-dodecanyl, n-tridecanyl, n-tetradecanyl, tetradecenyl, pentadecenyl, hexadecenyl, heptadecenyl, octadecenyl, nonadecenyl, eicosenyl, docosenyl, octadecadienyl, nonadecadienyl, eicosadienyl, octadecatrienyl, eicosatrienyl, eicosatetraenyl, octadecynyl, nonadecynyl, eicosynyl, and docosynyl. More preferred -R groups are selected from n-octanyl; n-nonanyl; n-decanyl; 9E- or 9Z-tetradecenyl; 9E- or 9Z-hexadecenyl; 9E- or 9Z-octadecenyl; 6E- or 6Z-octadecenyl; 11E-or 11Z-octadecenyl; 10E- or 10Z-nonadecenyl; 13E- or 13Z-docosenyl; 9-methylene-1-octadecanyl, 9Z; 12Z-octadecadienyl; 9E, 12E-octadecadienyl; 9Z, 12E-octadecadienyl; 9Z, 11E-octadecadienyl; 10E, 13E-nonadecadienyl; 11E, 14E-eicosadienyl; 9Z, 12Z, 15Z-octadecatrienyl; 6Z, 9Z, 12Z-octadecatrienyl; 11Z, 14Z, 17Z-eicosatrienyl; 5Z, 8Z, 11Z, 14Z-eicosatetraenyl; and 9-octadecynyl. Most preferred -R groups are n-octanyl, n-nonanyl, and 9Z-octadecenyl.

For the methods of the present invention which use vanillylamide or vanillylthioamide derivatives, particularly the beta-aminoethoxy-substituted compounds having the general structure:

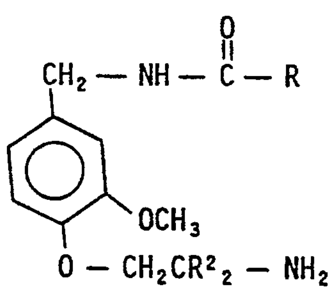,

the preferred -R groups are selected from n-hexanyl, n-heptanyl, n-octanyl, n-nonanyl, n-decanyl, n-undecanyl, n-dodecanyl, n-tridecanyl, tridecenyl, tetradecenyl, pentadecenyl, hexadecenyl, heptadecenyl, octadecenyl, nonadecenyl, eicosenyl, docosenyl, heptadecadienyl, octadecadienyl, nonadecadienyl, eicosadienyl, heptadecatrienyl, octadecatrienyl, nonadecatrienyl, eicosatrienyl, nonadecatetraenyl, heptadecynyl, octadecynyl, nonadecynyl, and eicosynyl. More preferred -R groups are selected from n-heptanyl; n-octanyl; n-nonanyl; 8E- or 8Z-tridecenyl; 8E- or 8Z-pentadecenyl; 8E- or 8Z-heptadecenyl; 5E- or 5Z-heptadecenyl; 10E- or 10Z-heptadecenyl; 9E- or 9Z-octadecenyl; 12E- or 12Z-nonadecenyl; 8-methylene-1-heptadecanyl; 8Z, 11Z-heptadecadienyl; 8E, 11E-heptadecadienyl; 8Z, 11E-heptadecadienyl; BZ, 10E-heptadecadienyl; 9E, 12E-octadecadienyl; 10E, 13E-nonadecadienyl; 8Z, 11Z, 14Z-heptadecatrienyl; 5Z, 8Z, 11Z-heptadecatrienyl; 10Z, 13Z, 16Z-nonadecatrienyl; 4Z, 7Z, 10Z, 13Z-nonadecatetraenyl; and 8-heptadecynyl. Most preferred -R groups are n-heptanyl, n-octanyl and 8Z-heptadecenyl (i.e., oleoyl amide).

For the methods of the present invention which use phenylacetic acid amide or thioamide derivatives, particularly compounds having the general structure:

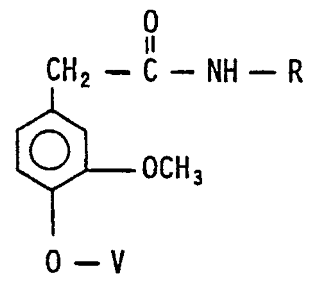

wherein -V is hydrogen or methyl; and the preferred -R groups are selected from n-hexanyl, n-heptanyl, n-octanyl, n-nonanyl, n-decanyl, n-undecanyl, n-dodecanyl, n-tridecanyl, n-tetradecanyl, tetradecenyl, pentadecenyl, hexadecenyl, heptadecenyl, octadecenyl, nonadecenyl, eicosenyl, docosenyl, octadecadienyl, nonadecadienyl, eicosadienyl, octadecatrienyl, eicosatrienyl, eicosatetraenyl, octadecynyl, nonadecynyl, eicosynyl, and docosynyl. More preferred -R groups are selected from n-octanyl; n-nonanyl; n-decanyl; 9E- or 9Z-tetradecenyl; 9E- or 9Z-hexadecenyl; 9E- or 9Z-octadecenyl; 6E or 6Z-octadecenyl; 11E- or 11Z-octadecenyl; 10E- or 10z-nonadecenyl; 13E- or 13Z-docosenyl; 9-methylene-1-octadecanyl, 9Z, 11E-octadecadienyl; 10E, 13E-nonadecadienyl; 11E, 14E-eicosadienyl; 9Z, 12Z, 15Z-octadecatrienyl; 6Z, 9Z, 12Z-octadecatrienyl; 11Z, 14Z, 17Z-eicosatrienyl; 5Z, 8Z, 11Z, 14Z-eicosatetraenyl; and 9-octadecynyl. Most preferred -R groups are n-octanyl, n-nonanyl, and 9Z-octadecenyl.

For the methods of the present invention which use vanillylamide or vanillylthioamide derivatives, particularly compounds having the general structure:

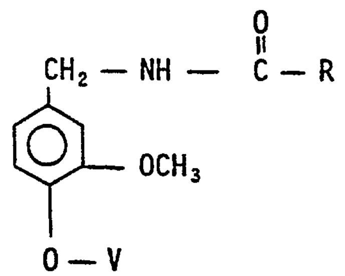

wherein -V is hydrogen or methyl; and the preferred -R groups are selected from n-hexanyl, n-heptanyl, n-octanyl, n-nonanyl, n-decanyl, n-undecanyl, n-dodecanyl, n-tridecanyl, tridecanyl, tetradecenyl, pentadecenyl, hexadecenyl, heptadecenyl, octadecenyl, nonadecenyl, eicosenyl, docosenyl, heptadecadienyl, octadecadienyl, nonadecadienyl, eicosadienyl, heptadecatrienyl, octadecatrienyl, nonadecatrienyl, eicosatrienyl, nonadecatetraenyl, heptadecynyl, octadecynyl, nonadecynyl, and eicosynyl. More preferred -R groups are selected from n-heptanyl; n-octanyl; n-nonanyl; 8E- or 8Z-tridecenyl; 8E- or 8Z-pentadecenyl; 8E- or 8Z-heptadecenyl; 5E- or 5Z-heptadecenyl; 10E- or 10Z-heptadecenyl; 9E- or 9Z-octadecenyl; 12E- or 12Z-nonadecenyl; 8-methylene-1-heptadecanyl; 8Z, 11Z-heptadecadienyl; 8E, 11E-heptadecadienyl; 8Z, 11E-heptadecadienyl; 8Z, 10E-heptadecadienyl; 9E, 12E-octadecadienyl; 10E, 13E-nonadecadienyl; BZ, 11Z, 14Z-heptadecatrienyl; 5Z, 8Z, 11Z-heptadecatrienyl; 10Z, 13Z, 16Z-nonadecatrienyl; 4Z, 7Z, 10Z, 13Z-nonadecatetraenyl; and 8-heptadecynyl. Most preferred -R groups are n-heptanyl, n-octanyl and 8Z-heptadecenyl (i.e., oleoyl amide).

The -R alkyl groups may be substituted or, preferably, unsubstituted. Preferred substituents are selected from the group consisting of halogen, hydroxy, amino, aryl, carboxylate, and $-OR^3$ wherein $-R^3$ is an unsubstituted alkyl group having from about 1 to about 3 carbon atoms (especially methoxy and ethoxy). It is preferred that substituted alkyl groups be mono-, di- or trisubstituted, most preferably monosubstituted.

Preferred compounds of the present invention include 8-methyl-N-vanillyl-6-nonenamide; N-vanillylnonanamide; N-vanillyl-9-octadecenamide; N-((4(2-aminoethoxy)-3-methoxyphenyl)methyl)-9Z-octadecenamide; N-((4-(2-aminoethoxy)-3-methoxyphenyl)methyl)-nonanamide; N-((4-(2-methyl-2-aminopropoxy)-3-methoxyphenyl)-methyl)9Z-octadecenamide; N-((4-(2-amino-3-methylbutoxy)-3-methoxyphenyl)-methyl)-9Z-octadecenamide; N-(9Z-octadecenyl)-4-(2-aminoethoxy)-3-methoxyphenylacetamide; N-octanyl-4(2-aminoethoxy)-3-methoxyphenylacetamide; N-((4-(2-amino-3-hydroxypropoxy)-3-methoxyphenyl)-methyl)-9Z-octadecenamide; N-((4-(2-amino-2-carboxyethoxy)-3-methoxyphenyl)-methyl) 9Z-octadecenamide; and the pharmaceutically-acceptable salts and amides thereof. Most preferred compounds useful in the methods of the present invention include 8-methyl-N-vanillyl-6-nonenamide; N-vanillylnonanamide; N-vanillyl-9-octadecenamide; N-((4-(2-aminoethoxy)-3-methoxyphenyl)-methyl)-9Z-octadecenamide; N-(9Z-octadecenyl)-4-(2-aminoethoxy)-3-methoxyphenylacetamide; N-((4-(2-aminoethoxy)-3-methoxyphenyl)-methyl)-nonanamide; and the pharmaceutically-acceptable salts and amides thereof.

As noted hereinbefore, capsaicin and a wide variety of other substituted phenyl compounds are known to have analgesic and anti-inflammatory activity. The natural and synthetic vanilloid compounds of the present invention are efficacious to help treat respiratory diseases or disorders and the attendant discomfort.

Some specific pharmaceutical compositions useful in this invention are described in the following U.S. Patents, all incorporated by reference herein: U.S. Patent No. 4,401,663, Buckwalter, et al, issued August 30, 1983; U.S. Patent No. 4,424,205, LaHann, et al, issued January 31, 1984; U.S. Patent No. 4,443,473, Buckwalter, et al, issued April 12, 1984; U.S. Patent No. 4,493,848, LaHann, et al, issued January 15, 1985. Representative pharmaceutical compositions useful in the methods of the present invention are provided in the non-limiting Examples provided hereinafter. Such pharmaceutical compositions preferably comprise one or more of the vanilloid compounds and a pharmaceutically acceptable carrier.

The term "pharmaceutically-acceptable carrier", as used herein, means one or more compatible solid or liquid filler diluents or encapsulating substances which are suitable for administration to a human or lower animal. The term "compatible", as used herein, means that the components of a pharmaceutical carrier are capable of being commingled with the vanilloid compounds and with each other, in a manner such that there is no interaction which would substantially reduce the pharmaceutical efficacy of the pharmaceutical composition under ordinary use situations. Pharmaceutically-acceptable carriers must, of course, be of sufficiently high purity and sufficiently low toxicity to render them suitable for administration to the human or lower animal being treated.

The pharmaceutically-acceptable carrier employed in the methods of the present invention is used at a concentration sufficient to provide a practical size to dosage relationship. The pharmaceutically-acceptable carriers, in total, may comprise from about 90% to about 99.9% by weight of the pharmaceutical compositions of the present invention, preferably from about 95% to about 99.95%, and more preferably from about 98% to about 99.9%.

Total single dosages of the vanilloid compounds present in pharmaceutical compositions useful herein are generally from about 1 ug to about 0.5 g. Preferred single dosages are from about 10 ug to about 50 mg; more preferred are from about 20 ug to about 5 mg; and most preferred are from about 0.1 mg to about 2 mg.

The choice of a pharmaceutically-acceptable carrier to be used in conjunction with the compounds of the present invention is largely determined by the way the compound is to be administered. Such methods include parenteral (especially subcutaneous), oral and topical including administration by inhalation, insufflation or direct nasal application.

The preferred methods of the present invention involve topical administration of the vanilloid compounds. These methods include, but are not limited to, administration by insufflator, pressurized insufflator, nebulizer, spray, drop, rinse, jelly, nasal aerosol, ointment formulation, cream, lotion, cotton pledget, gauge packtail, metered-dose nasal spray, metered-pump sprayer, metered dose aerosolized spray, fixed-volume aerosol spray, nasal spray emulsion, inhalation aerosol, and nebulizer aerosol.

The compounds of the present invention can be applied topically, as is, or compositions may be formulated for topical delivery. Suitable pharmaceutically-acceptable carriers for topical application include those suited for use as dispersions, emulsions, nebulae, powders and vapors. Dispersions include, for example, solutions, suspensions, and colloidal dispersions. Emulsions include, for example, systems containing two immiscible liquids in which one is dispersed, in the form of very small globules, throughout the other. Nebulae include, for example, oily liquid preparations that may be broken up into fine droplets. Powders include solid component forms. Vapors include solids or liquids converted into gaseous form.

Dispersions, emulsions, nebulae and powders may be formulated into aerosol compositions. Different regions of the respiratory tract may be targeted utilizing suitable aerosol compositions. These compositions may be composed of droplets or particles in a gas, using either gas and a powder or atomized bulk liquids. Aerosol compositions can be formulated into compositions with or without a propellant. The aerosol

compositions preferably comprise from 25 to 99%, preferably 90 to 95%, of a suitable propellant. Examples of such propellants include fluorocarbons such as dichloroflouromethane and dichlorotetraflouroethane, and hydrocarbons such as propane, butane and isobutane.

Dispersons, emulsions, nebulae, powders and vapors can be formulated into inhalant compositions. Inhalants can include finely powdered, liquid or vapor compositions carried by an air current into the respiratory passages. Components of inhalant compositions may include ethereal oils, highly volatile substances such as menthol and camphor, suitable surfactants/dispersing agents such as oleic acid, lecithin or sorbitan trioleate, water or other co-solvents.

Pressurized or unpressurized nebulizers of both aqueous solutions and dry powders, may be used for the administration of compounds and compositions of the present invention as inhalants. Delivery to more distal regions of the respiratory tract can be accomplished using nebulizers which distribute composition components in a finely divided cloud. Examples of such nebulizers include jet nebulizers, spinning nebulizers and ultrasonic nebulizers. Metered-dose inhalers may also be used. Composition formulations for metered-dose inhalers may include dimethyl ether-flourocarbons blended with other components that are solvents and propellants.

Insufflators may also be used for the administration of compounds and compositions of the present invention. The compounds or compositions are blown into the nasal, oral or respiratory passages.

Dispersions, emulsions, nebulae, and powders can be formulated into nasal or oral sprays, the compositions being administered in the form of a spray of small droplet or particle size by ejecting the composition into the nasal or oral cavity. These compositions may be primarily aqueous or aqueous-organic and may contain other components such as sympathomimetics, antihistamines, local anesthetics and aromatics, such as menthol.

Dispersions, emulsions, nebulae, and powders can be formulated for administration to the upper and lower respiratory tract by incorporating the compounds or compositions of the present invention with air or other gasses. The formulations comprising droplets or particles for administration to the upper respiratory tract are preferably of an aerodynamic size (mean mass diameter) of from about 0.1µm to about 100µm, more preferably from about 10µm to about 100µm, even more preferably from about 50µm to about 100µm. Formulations particularly suited for administration to the lower respiratory tract are made by incorporating the compounds or compositions of the present invention with air or other gases to produce respiratory formulations. The term "lower respiratory formulations" as used herein means compounds or compositions of the present invention dispersed in air or other gasses to form discrete units with an aerodynamic size (mean mass diameter) of equal to or less than about 10µm. Preferred lower respiratory formulations have an aerodynamic size of from about 0.1µm to about 10µm. More preferably, the lower respiratory formulations have an aerodynamic size of from about 0.5µm to about 7µm. Even more preferably, the lower respiratory formulations have an aerodynamic size of from about 1µm to about 5µm.

Dispersions, emulsions, nebulae and powders can be formulated into drops, gargles, rinses or jellies, lotions or creams by incorporating a safe and effective amount of a vanilloid compound or composition with a pharmaceutically-acceptable solubilizing and/or dispersing agent (such as polysorbates, Pluronics®, Brijs®, polyvinylpyrrolidone, phospholipids, alkyl sulfates, etc.) and water; and/or pharmaceutically-acceptable co-solvents such as ethanol, propylene glycol, glycerin and PEG. Preservatives such as benzalkonium chloride and alkyl parabens and the like; and thickening agents such as modified cellulose gums of carboxyvinyl polymers, can be included as optional ingredients.

Lozenges or pastilles can be formulated by incorporating a safe and effective amount of a vanilloid compound or composition of the present invention with a hard candy base or as a compressed lozenge using pharmaceutically-acceptable binders and adhesives. Other acceptable components may include suitable flavoring and coloring agents to satisfy aesthetic requirements.

Syrups and elixirs can be formulated by incorporation of a safe and effective amount of a vanilloid compound or composition of the present invention with a syrup base. Optional ingredients may also include pharmaceutically-acceptable co-solvents such as ethanol, propylene glycol, glycerin and PEG and suitable flavoring and coloring agents.

## Methods of Treating Respiratory Tract Diseases or Disorders

The present invention provides methods of treating respiratory diseases or disorders in humans or lower animals comprising topically administering in a human or lower animal, with a respiratory disease or disorder, a safe and effective amount of a vanilloid compound or composition as disclosed herein.

"Topically administration", or "topically administering", as used herein, means contacting the epithelial

tissue of the respiratory tract with a safe and effective amount of a vanilloid compound or composition as disclosed herein, for the treatment of respiratory diseases or disorders.

The present invention further provides methods of treating respiratory diseases or disorders, including methods of alleviating signs and symptoms associated with respiratory tract infections and the attendant hypersecretion of mucus associated with various respiratory diseases or disorders. The methods of the present invention may be useful for preventing or treating recurrent respiratory episodes and/or for relieving the symptoms associated with hypersecretion of mucus in the respiratory tract. The methods of the present invention may further be useful to treat or prevent other respiratory disorders and diseases such as extrinsic, or especially intrinsic asthma.

The phrase "safe and effective amount", as used herein, means an amount of a compound or composition high enough to significantly positively modify the condition to be treated, but low enough to avoid serious side effects (at a reasonable benefit/risk ratio), within the scope of sound medical judgment. The safe and effective amount of the compound or composition will vary with the particular condition being treated, the age and physical condition of the patient being treated, the severity of the condition, the duration of the treatment, the nature of concurrent therapy, the specific compound or composition employed, the particular pharmaceutically-acceptable carrier utilized, and like factors within the knowledge and expertise of the attending physician. Daily dosages can range from about 0.1 mg/kg of body weight to about 10 mg/kg of body weight. Preferred daily dosages are from about 0.5 to about 2 mg/kg of body weight. Up to about 6 single dosages per day may be administered.

Topical administration can be accomplished by exposing the respiratory mucosa to a safe and effective amount of a vanilloid composition on the epithelial tissue, including oral, gingival, nasal, pharyngeal, tracheal, bronchial, and other respiratory tract tissue. The amount of the pharmaceutical composition to be administered may vary from about 0.05 $mg/cm^2$ to about 100 $mg/cm^2$, depending upon such factors as the sensitivity, type and location of tissue to be treated, the composition and carrier to be administered, and the particular compound to be administered. Preferred amounts of the pharmaceutical composition to be administered may vary from about 0.5 $mg/cm^2$ to about 10 $mg/cm^2$; even more preferred from about .1 $mg/cm^2$ to about 5 $mg/cm^2$; most preferred from about .2 $mg/cm^2$ to about 2.0 $mg/cm^2$.

Methods for synthesizing beta-aminoethoxy-substituted vanilloids used in the compositions of the present invention are described in EPA 0282127, assigned to The Procter & Gamble Company, published September 14, 1988, which is hereby incorporated by reference in its entirety.

Compositions for topical (regional) administration are prepared as follows:

Example 1

| Composition for Oral Inhalation Aerosol via Metered-Dose Dispenser | |
|---|---|
| Trans-8-methyl-N-vanillyl-6-nonenamide | 0.075 g |
| Sorbitan trioleate | 0.125 g |
| Trichloromenofluoromethane | 2.30 g |
| Dichlorotetrafluoroethane | 2.30 g |
| Dichlorodifluoromethane | 5.20 g |
| | 10.000 g |

The nonenamide is mixed with the other above-listed ingredients for use as an aerosol using methods known in the art. Each actuation delivers about 200 mcg of the nonenamide.

N-vanillylnonamide or other vanilloids useful in the compositions of the present invention may be substituted for the above nonenamide in the composition.

EXAMPLE II

| Composition for Oral Inhalators Aerosol via Metered-Dose Dispenser | |
|---|---|
| Trans-8-methyl-N-vanillyl-6-nonenamide | 0.025 g |
| Ascorbic acid | 0.010 g |
| Ethanol | 3.265 g |
| Dichlorodifluoromethane | 3.350 g |
| Dichlorotetrafluoromethane | 3.350 g |
| | 10.000 g |

The nonenamide is mixed with the other above-listed ingredients for use as an aerosol using methods known in the art. Each actuation delivers about 125 mcg of the nonenamide.

N-vanillylnonamide or other vanilloids useful in the compositions of the present invention may be substituted for the above nonenamide in the composition.

EXAMPLE III

| Composition for Nasal Spray Inhalation via Metered-Dose Manual Pump Spray | |
|---|---|
| N-vanillylnonanamide | 0.010 g |
| Benzalkonium chloride | 0.015 g |
| Polysorbate 80 | 0.030 g |
| Dextrose | 0.045 g |
| Carboxymethyl cellulose sodium | 0.050 g |
| Microcrystalline cellulose | 0.050 g |
| Water, q.d. | 8.80 g |
| | 10.000 g |

The nonanamide is mixed with the other above-listed ingredients for use as a nasal spray using methods known in the art. Each actuation delivers about 100 mg of suspension containing 100 mg of the nonanamide.

Trans-8-methyl-N-vanillyl-6-nonenamide or other vanilloids useful in the composition of the present invention may be substituted for the nonamide in the composition.

While particular embodiments of the present invention have been described, it will be obvious to those skilled in the art that various changes and modifications to the compounds and compositions disclosed herein can be made without departing from the spirit and scope of the invention. It is intended to cover, in the appended claims, all such modifications that are within the scope of this invention.

## Claims

1. Use of a compound for the manufacture of a topical medicament for treating respiratory diseases or disorders in humans or lower animals characterized in that the compound has the general structure:

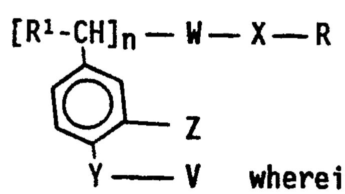

wherein:

a) n is 0 or 1, preferably n is 1;

b) -W-X- is selected from -C(O)NH-, -C(S)NH-, $-S(O)_2NH-$, -NHC(O)O-, -NHC(S)O-, -NHC(O)NH- and -NHC(S)NH-, preferably from -C(O)NH-, -C(S)NH-, -NHC(O)NH-, -NHC(S)NH-, and $-S(O)_2NH-$, more preferably from -C(O)NH- and -C(S)NH-; where either available bond of -W-X- is bonded to -R and the other bond is attached to the benzyl carbon atom or to the benzene ring;

c) $-R^1$ is selected from hydrogen, hydroxy, alkyl esters of hydroxy having from 1 to 5 carbon atoms, alkyl having from 1 to 5 carbon atoms, and alkoxy having from 1 to 5 carbon atoms, preferably from -H, -OH and $-CH_3$, more preferably $-R^1$ is -H;

d) -Z is selected from -H, -OH, $-OCH_3$, preferably from -OH and $-OCH_3$;

e) -Y- is selected from -O-, -S-, $-NR^4-$, -OC(O)-, $-OSO_3^{-}-$, and $-OPO_3^{=}-$, where $-R^4$ is selected from hydrogen and $C_1$-$C_4$ alkanyl, preferably Y is selected from -NH-, -O- and -S-, more preferably -Y- is -O-;

f) -V is selected from -H, short chain alkyl, and $-CR^2_2$- $CR^2_2$ $-NH_2$;

g) $-R^2$ moieties are independently selected from hydrogen; halogen; unsubstituted or substituted alkyl, the alkyl portion having from 1 to 6 carbon atoms; substituted or unsubstituted aryl; and carboxylate; or two $-R^2$ moieties are covalently bonded to form a substituted or unsubstituted alkyl, heteroalkyl, aryl or heteroaryl ring having from 3 to 8 atoms in the ring including from 0 to 3 heteroatoms, preferably no more than two $R^2$'s are other than hydrogen; and

h) -R is $C_1$-$C_{24}$ alkyl, preferably -R is selected from unsubstituted, saturated, or mono- or di-unsaturated, $C_6$-$C_{24}$ straight or branched chain alkyl, or arylalkyl having a $C_1$-$C_{12}$ alkyl portion, more preferably -R is a mono-unsaturated, cis double bond, $C_{11}$-$C_{23}$ straight chain alkenyl.

2. The use of Claim 1 characterized in that -V is selected from -H and $-CH_3$.

3. The use of Claim 1 characterized in that the compound is trans-8-methyl-N-vanillyl-6-nonenamide or N-vanillylnonanamide.

4. The use of Claim 1 characterized in that preferably -Z is $-OCH_3$; -V is $-CR^2_2$-$CR^2_2$-$NH_2$; preferably all $-R^2$'s are selected from hydrogen, unsubstituted or substituted $C_1$-$C_6$ alkyl, unsubstituted or substituted phenyl, and two $-R^2$'s being bonded to form a substituted or unsubstituted $C_3$-$C_6$ alkyl ring; more preferably all $-R^2$'s are -H or both $-R^2$'s on the beta carbon are selected from methyl and ethyl or are covalently bonded to form cyclopropyl, cyclobutyl or cyclopentyl; and preferably -R is selected from saturated or mono-unsaturated, $C_6$-$C_{24}$ straight or branched chain alkyl, more preferably -R is selected from mono-unsaturated or saturated, $C_6$-$C_{10}$ straight chain alkyl, and mono-unsaturated, cis-double bond, $C_{14}$-$C_{23}$ straight chain alkenyl.

5. The use of Claim 1 characterized in that the compound is selected from N-(9Z-octadecenyl)-4-(2-aminoethoxy)-3-methoxyphenylacetamide; N-octanyl-4-(2-aminoethoxy)-3-methoxyphenylacetamide; N-((4-(2-aminoethoxy)-3-methoxyphenyl)-methyl)-9Z-octadecenamide; and N-((4-(2-aminoethoxy)-3-methoxyphenyl)methyl)-nonanamide.

6. The use of any of Claims 1-5 characterized in that the compound is used to treat respiratory diseases or disorders selected from intrinsic asthma, chronic bronchitis, or diseases of the extrathoracic (upper) airways caused by viral infection.

7. The use of any of Claims 1-5 characterized in that the compound is used to treat respiratory diseases or disorders selected from allergic rhinitis and vasomotor rhinitis.

8. The use of any of Claims 1-5 characterized in that the compound is used to treat respiratory diseases or disorders selected from laryngitis and pharyngitis.

9. A pharmaceutical composition for topical administration to the respiratory passageway of a human or lower animal characterized in that it comprises a compound of discrete liquid or solid particles, having an aerodynamic size of from 0.1 µm to 100 µm, preferably from 50 µm to 100 µm, dispersed in a gas phase, said particles comprising a compound having the general structure:

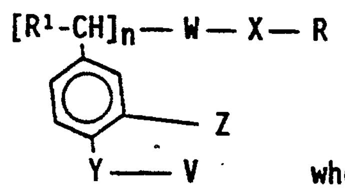

wherein:

a) n is 0 or 1, preferably n is 1;

b) -W-X- is selected from -C(O)NH-, -C(S)NH-, $-S(O)_2NH-$, -NHC(O)O-, -NHC(S)O-, -NHC(O)NH- and -NHC(S)NH-, preferably from -C(O)NH- and -C(S)NH-; where either available bond of -W-X- is bonded to -R

and the other bond is attached to the benzyl carbon atom or to the benzene ring;

c) -$R^1$ is selected from hydrogen, hydroxy, alkyl esters of hydroxy having from 1 to 5 carbon atoms, alkyl having from 1 to 5 carbon atoms, and alkoxy having from 1 to 5 carbon atoms, preferably -$R^1$ is selected from -H, -OH and -$CH_3$, more perferably -$R^1$ is -H;

d) -Z is selected from -H, -OH, -$OCH_3$, preferably from -OH and $OCH_3$;

e) -Y- is selected from -O-, -S-, -$NR^4$-, -OC(O)-, -$OSO_3{}^=$-, and -$OPO_3{}^=$-, where -$R^4$ is selected from hydrogen and $C_1$-$C_4$ alkanyl, preferably -Y- is -O-;

f) -V is selected from -H, short chain alkyl, and -$CR^2{}_2$- $CR^2{}_2$ -$NH_2$, preferably from -H and -$CH_3$;

g) -$R^2$ moieties are independently selected from hydrogen; halogen; unsubstituted or substituted alkyl, the alkyl portion having from 1 to 6 carbon atoms; substituted or unsubstituted aryl; and carboxylate; or two -$R^2$ moieties are covalently bonded to form a substituted or unsubstituted alkyl, heteroalkyl, aryl or heteroaryl ring having from 3 to 8 atoms in the ring including from 0 to 3 heteroatoms, preferably no more than two -$R^2$'s are other than hydrogen; and

h) -R is $C_1$-$C_{24}$ alkyl, preferably -R is selected from saturated or mono- or di-unsaturated with double bonds $C_6$-$C_{24}$ straight or branched chain alkyl, more preferably from monounsaturated, cis double bond, $C_{11}$-$C_{23}$ straight chain alkenyl.

10. A pharmaceutical composition according to Claim 9 characterized in that -V is -$CR^2{}_2$-$CR^2{}_2$-$NH_2$, preferably all -$R^2$'s are selected from hydrogen, unsubstituted or substituted $C_1$-$C_6$ alkyl, unsubstituted or substituted phenyl, and two -$R^2$'s being bonded to form a substituted or unsubstituted $C_3$-$C_6$ alkyl ring, more preferably all -$R^2$'s are -H or both -$R^2$'s on the beta carbon are selected from methyl and ethyl or are covalently bonded to form cyclopropyl, cyclobutyl or cyclopentyl.